(19) Europäisches Patentamt
European Patent Office
Office européen des brevets

(11) **EP 1 767 170 A2**

(12) **EUROPÄISCHE PATENTANMELDUNG**

(43) Veröffentlichungstag:
**28.03.2007 Patentblatt 2007/13**

(51) Int Cl.:
*A61F 2/32* (2006.01)     *A61F 2/30* (2006.01)
*B24B 19/02* (2006.01)

(21) Anmeldenummer: 06023817.7

(22) Anmeldetag: **06.03.1997**

(84) Benannte Vertragsstaaten:
**AT CH DE FR GB IT LI**

(30) Priorität: **12.04.1996 EP 96810225**

(62) Dokumentnummer(n) der früheren Anmeldung(en)
nach Art. 76 EPÜ:
**03007042.9 / 1 340 477**
**97903208.3 / 0 892 627**

(71) Anmelder: **Zimmer GmbH**
**8404 Winterthur (CH)**

(72) Erfinder: **Semlitsch, Manfred**
**8400 Winterthur (CH)**

(74) Vertreter: **Manitz, Finsterwald & Partner GbR**
**Postfach 31 02 20**
**80102 München (DE)**

Bemerkungen:
Diese Anmeldung ist am 16 - 11 - 2006 als
Teilanmeldung zu der unter INID-Kode 62 erwähnten
Anmeldung eingereicht worden.

(54) **Verfahren zur Herstellung einer Lagerschale und einer Gelenkkugel eines künstlichen Gelenks**

(57) Die Erfindung betrifft eine Lagerschale (1) für ein künstliches Gelenk, insbesondere künstliches Hüftgelenk, die eine konkave sphärische Fläche A mit Mittelpunkt Ms aufweist, wobei die Lagerschale (1) aus einem verschleißfesten metallischen Werkstoff besteht und der Formfehler der Fläche A über einen Winkel 90° < $\alpha$ < 180° weniger als plus/minus 7,5 $\mu$m beträgt, während die Rauhigkeit der Fläche A einem Wert $R_a$ < 0,08 $\mu$m entspricht.

Fig. 1

EP 1 767 170 A2

**Beschreibung**

Die Erfindung bezieht sich auf ein Verfahren zum Herstellen einer Lagerschale eines künstlichen Gelenks, die eine konkave sphärische Fläche A mit einem Radius R aufweist, oder zum Herstellen einer Gelenkkugel eines künstlichen Gelenks, die eine konvexe sphärische Fläche B mit einem Radius r aufweist, mit den weiteren Merkmalen des Anspruchs 1.

Künstliche Gelenke erfordern Materialpaarungen der sich gegeneinander bewegenden Lagerkörper, die gute Notlaufeigenschaften aufweisen. Der klassische Ansatz in der Materialkombination besteht daher auch in der Paarung ungleicher Partner. So werden relativ weiche Lagerschalen aus Polyäthylen mit harten Gelenkköpfen aus Metall oder Keramik kombiniert und in den Anfängen der künstlichen Hüftgelenke metallische Werkstoffe mit unterschiedlicher Härte und Verschleißfestigkeit miteinander kombiniert. Trotz aller Anstrengungen konnte mit diesen Werkstoffkombinationen der Verschleiß der Partner nie ganz eliminiert werden. Bei Polyäthylen findet beispielsweise im Hüftgelenk ein Abrieb statt, durch welchen die Lagerfläche jährlich um ca. 0,2 mm in Richtung der Hauptkraft zurückgesetzt wird und auch bei metallischen Flächen treten durch Punktlasten und Mikroverschweißungen in den Oberflächen Verschleißerscheinungen auf, die - wenn sie einmal angefangen haben - sehr schnell die ganze Eingriffsfläche in Mitleidenschaft ziehen.

In der Publikation STREICHER: "Untersuchung des tribologischen Verhaltens von Metall/Metall Kombinationen für künstliche Hüftgelenke" (BIOMEDIZINISCHE TECHNIK, Bd. 35, Nr. 5, 1990, Berlin, DE, S. 107-111) werden Versuchsanordnungen beschrieben, die eine recht genaue Verschleißmessung bei Verschleißversuchen an künstlichen Hüftgelenken erlauben. Untersucht wurde der Verschleiß zwischen gleichartigen CoCrMo-Legierungen und CoCrMo-Legierung gegen UHMWPE als Referenz. Es wurden Paarungen mit unterschiedlichem Spiel zwischen Kugel und Pfanne untersucht. Die Auswertung der Versuche erfolgte durch permanente Aufnahme des Reibungsmomentes sowie durch periodisches Ausmessen der Implantatskomponenten. Außerdem wurde die Methode der Dünnschichtaktivierung angewendet, um auch kleine Verschleißraten feststellen zu können. Die Auswertung zeigt nur eine geringe Abhängigkeit der Reibung von der Gleitgeschwindigkeit, sowie ein höheres Reibmoment der CoCrMo-Gußlegierung gegen sich selbst im Vergleich gegen Polyethylen. Eine Reduktion des Lagerdurchmessers in den Bereich eines Lagers mit Polymer/Metallpaarung ergab für die Paarung von CoCrMo-Schmiedelegierungen ähnliche Reibungsverluste wie bei der Polymer/Metallpaarung. Für den Verschleiß wird bei gleichem Spiel der Einfluss des Lagerdurchmessers aufgezeigt. Außerdem wird gezeigt, dass das Reibmoment bei einer Metall/Metall Paarung nach einer Einlaufphase mit höherem Reibmoment zurückgehen kann.

Nicht gezeigt ist, welche Anfangsgeometrie notwendig ist, um zu verschleißarmen Paarungen zu kommen, noch sind Verfahren aufgezeigt, die zu einer Verbesserung der Situation führen.

Ein künstliches Gelenk mit metallischen Lagerflächen, das bestimmten geometrischen Bedingungen genügt, ist aus der WO 97/16138 A1 bekannt. Dieses Dokument fällt unter Art. 54(3) EPÜ.

Aufgabe der Erfindung ist es, eine verschleißarme Anordnung zu erreichen.

Die Lösung dieser Aufgabe erfolgt durch die Merkmale des Anspruchs 1 und insbesondere dadurch, dass die Lagerschale als Werkstück als eine mit Untermass im Bereich der Lagerfläche A vorgedrehte Schale mit ihrer Polachse in der Rotationsachse einer Werkzeugmaschinenspindel aufgespannt wird, oder dass die vorgedrehte Gelenkkugel als Werkstück mit Übermaß im Bereich der Lagerfläche B mit ihrer Aufspannachse D in der Rotationsachse einer Werkzeugmaschinenspindel aufgespannt wird, und dass während dem Rotieren des Werkstücks ein kreiszylindrischer Schleifkörper, der mit seiner Zylinderachse in der Rotationsachse einer Werkzeugspindel rotierend aufgespannt ist, mit einer kreisförmigen Kante seiner Stirnseite unter Zugabe von Schleifmittel an die Lagerfläche A, B des Werkstücks angepresst wird, wobei sich die Rotationsachse der Werkzeugspindel in einem Auslenkwinkel $\gamma$, $\delta$ < 90° mit der Rotationsachse der Werkstückspindel in einem Schnittpunkt schneidet, und wobei der Anpressdruck durch Zustellen der Werkzeugspindel in der Richtung ihrer Rotationsachse erfolgt.

Durch dieses Verfahren lässt sich erreichen, dass Lagerschale und Gelenkkopf aus einem verschleißfesten metallischen Werkstoff bestehen, dass die Fläche A einen mittleren Radius $R_m$ und die Fläche B einen mittleren Radius $r_m$ aufweisen, wobei deren Differenz 35 $\mu$m < $R_m$-$r_m$ < 85 $\mu$m beträgt, dass der Formfehler der Fläche A über einen Winkel 90° < $\alpha$ < 180° weniger als plus/minus 7,5 $\mu$m beträgt, dass der Formfehler der Fläche B über einen Winkel $\beta$ > 140° weniger als plus/minus 2 $\mu$m beträgt, und dass die Gelenkkugel außerhalb der Fläche B durch eine zurückgesetzte Fläche C fortgesetzt ist, die einen geringeren Abstand zum Mittelpunkt $M_K$ als die Fläche B aufweist, während die Rauhigkeit der Fläche A einem Wert $R_a$ < 0,08 $\mu$m und die Rauhigkeit der Fläche B einem Wert $R_a$ < 0,08 $\mu$m entspricht.

Durch das Herstellen, Vermessen und Paaren von Lagerflächen aus einem gleichartigen verschleißfesten, metallischen Werkstoff wird zwischen den Lagerflächen eine Geometrie erreicht, die zusammen mit der Kapillarwirkung der Körperflüssigkeit und dem Auftrieb bei aneinander vorbei gleitenden Lagerflächen Mikroverschweißungen und Verschleiß weitgehend verhindert. Durch das Unterdrücken der Mikroverschweißungen können bei gleichartigen, verschleißfesten metallischen Werkstoffen, die positiven Eigenschaften dieser Werkstoffe wie Zähfestigkeit, Formbeständigkeit und Elastizität genutzt werden. Es entstehen Oberflächen an einem homogenen Gefüge, die weder wegen Härteunterschieden zwischen Oberfläche und Grundkörper bei starker Belastung durchbrochen werden noch sich vom Grundkörper ablösen. Gleichzeitig sind die Flächen so gut aneinander angeglichen, dass keine unzulässige Pressung im Stillstand auftritt.

Diese Wirkung wird noch besser, wenn die Rauhigkeit der Flächen A und B einem Wert $R_a < 0,05\ \mu m$ entspricht.

Besonders geeignet sind Werkstoffe wie Kobalt-, Chrom-, Nickellegierungen wie beispielsweise nach ISO 5832/4 das Material PROTASUL 21 WF der Firma SULZER AG, wenn sie nach einem Verfahren gefertigt werden, bei dem eine kreisförmige Erzeugende für die Kugelform ebenfalls dreht, jedoch in ihrer Drehachse zur Drehachse des Werkstücks verschwenkt ist, um eine Kugelform zu schleifen, zu honen und zu polieren bis die vorgegebenen Toleranzen für Durchmesser, Formgenauigkeit und Oberflächengüte erreicht sind.

Lösbare Verbindungen für Innenschalen sind durch die Verwendung von Innenschalen aus relativ elastischem Polyäthylen bekannt. Diese lassen sich nicht von Innenschalen aus wesentlich zähfesteren metallischen Werkstoffen übernehmen. Funktion und Herstellung sprechen dagegen. Es ist daher sinnvoll, die Lagerschale an ihrer Außenseite fest mit einem Zwischenkörper aus elastischem Kunststoff wie beispielsweise Polyäthylen zu verbinden, der seinerseits mit einer äußeren Schale lösbar verbunden werden kann. Insbesondere können auch implantierte Kunststoffschalen durch die metallische Lagerschale ersetzt werden, wenn die lösbare Verbindung des Zwischenkörpers die gleiche ist, und ebenso können die von der Präzision her notwendigen Gelenkkugeln an Prothesenschäften ausgewechselt werden, wenn die Gelenkkugeln über eine lösbare Verbindung, zum Beispiel über eine lösbare Konusverbindung, auf dem Schaft verfügen. Dadurch, dass Gelenkkopf und Lagerschale auswechselbar gestaltet sind, können sie steril verpackt bis auf den Operationstisch gebracht werden. Die Lagekontrolle eines implantierten Gelenks erfolgt noch mit Manipuliergelenkkugeln, die die Schalen nicht verletzen, während die Präzisionskugeln erst gegen Schluss eingesetzt werden.

Interessanterweise hat sich gezeigt, dass für eine verschleißarme Paarung Metall/Metall bei vorgegebener Rauhigkeit Ra die Formfehler der einzelnen Teile für die Schmierung eine größere Rolle spielen als die Bandbreite, innerhalb welcher die Differenz der mittleren Radien $R_m\text{-}r_m$ liegen darf. Bei einem für künstliche Hüftgelenke üblichen Kugeldurchmesser beispielsweise für einen Nenndurchmesser von 28 mm darf die Differenz der mittleren Radien $35\ \mu m < R_m\text{-}r_m < 85\ \mu m$ betragen, was einer Bandbreite von $50\ \mu m$ entspricht, wenn man von absoluten angestrebten Herstellmassen und nicht von Auswahlpaarungen ausgeht. Bei einer Halbierung dieses Bandes würde für beide Teile eine absolute Herstellgenauigkeit von $25\ \mu m$ für den mittleren Radius $R_m$

oder $r_m$ verbleiben. Diese Werte sind gross genug, um auf eine Auswahlpaarung verzichten zu können und somit jede Kugel mit jeder Lagerschale paaren zu können. Dies ist jedoch nur möglich, wenn die Formgenauigkeit beherrscht wird. Diese ist sehr genau einzuhalten und bedarf besonderer Herstellmethoden, um die erforderlichen Toleranzwerte einzuhalten.

Mit der Erfindung ist ein für sich neues und erfinderisches Herstellungsverfahren aufgezeigt, wonach die Lagerschale als Werkstück als eine mit Untermass im Bereich der Lagerfläche A vorgedrehte Schale mit ihrer Polachse in der Rotationsachse einer Werkzeugmaschinenspindel aufgespannt wird oder wonach die vorgedrehte Gelenkkugel als Werkstück mit Übermaß im Bereich der Lagerfläche B mit ihrer Aufspannachse D in der Rotationsachse einer Werkzeugmaschinenspindel aufgespannt wird und wonach während dem Rotieren des Werkstücks ein kreiszylindrischer Schleifkörper, der mit seiner Zylinderachse in der Rotationsachse einer Werkzeugspindel rotierend aufgespannt ist, mit einer kreisförmigen Kante seiner Stirnseite unter Zugabe von Schleifmittel, an die Lagerfläche A, B des Werkstücks angepresst wird, wobei sich die Rotationsachse der Werkzeugspindel in einem Auslenkwinkel $\gamma$, $\delta$ kleiner als 90° mit der Rotationsachse der Werkstückspindel in einem Schnittpunkt schneidet und wobei der Anpressdruck durch Zustellen der Werkzeugspindel in der Richtung ihrer Rotationsachse erfolgt.

Diese Anordnung hat den Vorteil, dass sich Werkzeug und Werkstück - im Rahmen der Steifigkeit der Spindeln an denen sie eingespannt sind - an den Arbeitsflächen gegenseitig zentrieren. Aufgrund der Ablaufbewegung entsteht an Werkstück und an Werkzeug ein Verschleiß, der zwangsläufig zur Bildung einer Kugelfläche an beiden Teilen führt. Am Werkzeug entsteht an einer gebrochenen Kante an der Stirnseite ein schmales kreisförmiges Band einer Kugelfläche, während am Werkstück für die gleiche Kugelform Kugelflächen A, B entstehen. Dadurch, dass jeder Punkt der Werkzeugarbeitsfläche gegenüber jedem Punkt der Bearbeitungsfläche in Eingriff kommt, entstehen perfekte Ausschnitte A, B. von Kugelflächen.

Die abhängigen Ansprüche stellen vorteilhafte Weiterbildungen des erfindungsgemäßen Verfahrens dar. So ist es für die Herstellung der Lagerfläche A einer Lagerschale vorteilhaft, den Winkel zwischen Rotationsachsen von Lagerschale und Werkzeug so zwischen 39° und 45° zu wählen, dass ein möglichst großer Begrenzungswinkel $\alpha$ für die Lagerfläche entsteht, weil dann der Durchmesser für die Erzeugende und einen ihr entsprechenden Zylinder so groß gewählt werden kann, dass der Begrenzungswinkel $\alpha$ gegen 180° wachsen kann, ohne dass dieser Zylinder den Schaleninnenrand touchiert. Es ist somit nur eine geführte Zustellbewegung in der Richtung der Rotationsachse des Werkzeugs notwendig, um zu einem großen Begrenzungswinkel $\alpha$ zu kommen. Bei einem Begrenzungswinkel $\alpha$ der um einen beträchtlichen Betrag kleiner als 180° ist, können zwar größere Kreisdurchmesser für eine Erzeugende gewählt werden, hingegen ist die Erzeugende dann nur noch als unterbrochener Kreis im Eingriff.

Bei der Herstellung der Gelenkkugel hat sich gezeigt, dass eigentlich schon eine Lagerfläche B mit einem Begrenzungswinkel β von etwa 180° bei einer Formgenauigkeit von plus/minus 2 μm für die Funktion des Lagers genügt, sofern einerseits alle anderen Flächenteile an der Gelenkkugel weiter zurückstehen und andererseits im Zustand der Normalbelastung der Äquator von Lagerschale und von Lagerfläche B annähernd parallel zueinander ausgerichtet sind. Der Auslenkwinkel γ für ein rotierendes Werkzeug mit einem kreiszylindrischen Hohlzylinder kann in grö-ßeren Grenzen beispielsweise zwischen 60° bis 20° eingestellt werden, um eine Lagerfläche B zu erzeugen. Da die Erzeugende Kreisringfläche auch bei einem Begrenzungswinkel β von mehr als 180° nicht bis an den Mittelpunkt der Lagerfläche B herankommt, kann der Durchmesser der Gelenkkugel über die Lagerfläche B während der Bearbeitung, beispielsweise durch einen Taster mit diamantbestückten Abtastflächen, abgefragt werden, um die restliche Bearbeitungszeit genügend genau zu extrapolieren.

Abgesehen vom Polieren eines Einlaufradius am Äquator der Lagerfläche A der Lagerschale können die anderen Arbeitsgänge für die erforderliche Präzision von Lagerschale und Gelenkkugel auf einer numerisch gesteuerten Werkzeugmaschine automatisch durchgeführt werden.

Der geringe Verschleiß an Kugel und Lagerschale eröffnet im Weiteren den Vorteil, dass mit der erfindungsgemäßen Ausführung keine Reoperation wegen sich verschlechternder Lagerflächen notwendig ist. Aus diesem Grund können die Gelenkkugeln bei zementierten Prothesenschäften sogar einstückig mit dem Schaft verbunden sein und bei direkt eingesetzten Schäften, z.B. aus Titan, mit einer Einmal-Verbindung befestigt werden, solange die Operationstechnik nicht aus Platzgründen eine nachträgliche Befestigung der Kugel am implantierten Schaft vorschreibt. Insbesondere lohnt es sich anatomisch angeglichene und fest verankerbare Schäfte, die eine S-förmige Schaftform mit einer Anteversion vom proximalen Halsbereich und mit einem gegen posterior in einer Krümmung oder in einem Knick wegstehenden Schaftende besitzen, einzusetzen, da nur noch die Dauer der Verankerung den Termin für einen weiteren Eingriff bestimmt.

Im Folgenden wird die Erfindung anhand von Ausführungsbeispielen beschrieben. Es zeigen:

Fig. 1 schematisch einen Längsschnitt durch eine Lagerschale;

Fig. 2a schematisch einen Schnitt durch eine Gelenkkugel mit einem Begrenzungswinkel β > 180°;

Fig. 2b schematisch einen Schnitt durch eine Gelenkkugel mit einem Begrenzungswinkel β < 180°;

Fig. 3 schematisch und abgewickelt den Profilschrieb einer Lagerschale und den einer Gelenkkugel, die im Abstand ihrer mittleren Radien zueinander angeordnet sind;

Fig. 4 schematisch eine Anordnung von Gelenkkugel und Werkzeug bei der Herstellung auf einer Werkzeugmaschine;

Fig. 5 schematisch eine Anordnung von Lagerschale und Werkzeug bei der Herstellung auf einer Werkzeugmaschine; und

Fig. 6 schematisch die Ausführung einer Lagerschale, welche mit einem Kunststoffzwischenkörper fest verbunden ist, der seinerseits lösbar in einer äußeren Schale befestigt ist.

Mit den Figuren wird eine Geometrie beschrieben, die es ermöglicht, gleichartige, verschleißfeste, metallische Werkstoffe wie PROTASUL 21 WF als Lagerschale 1 und Gelenkkugel 2 in einem sphärischen Lager zu verwenden, ohne dass Mikroverschweißungen und exzessiver Verschleiß auftreten, während andere Eigenschaften wie Zähfestigkeit, Formbeständigkeit und Elastizität für die Funktion genutzt werden können. Durch eine passende Relation zwischen den mittleren Radien $R_m$, $r_m$ der Lagerflächen A und B sowie der zulässigen Formabweichungen 12, 13 und der zulässigen Rauhigkeit der Lagerflächen werden Mikroverschweißungen des gleichartigen metallischen Werkstoffs weitgehend verhindert.

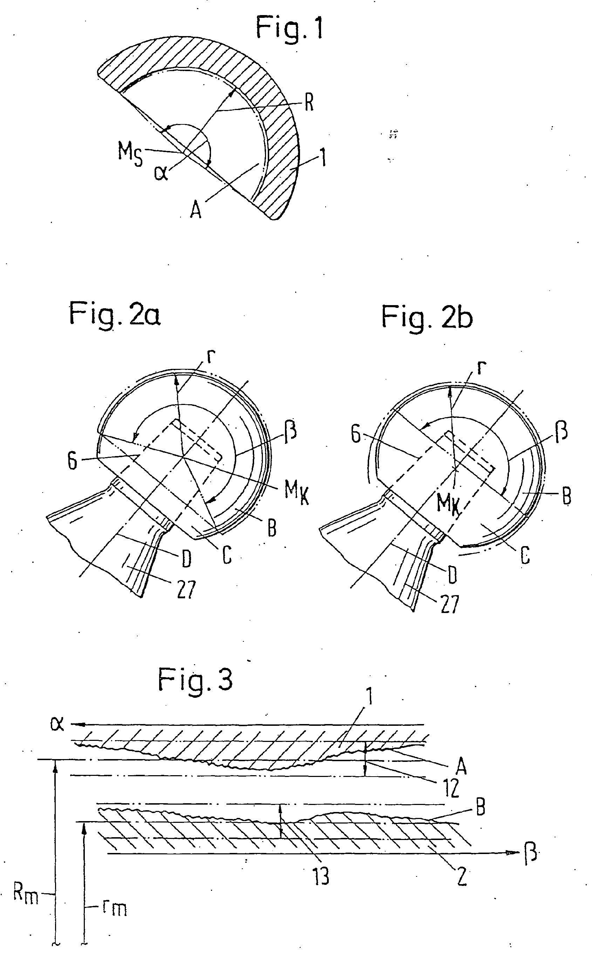

In Figur 1 ist eine Lagerschale 1 für ein künstliches Hüftgelenk dargestellt, deren sphärische Lagerfläche A sich über einen Winkel α von etwas weniger als 180° erstreckt und einen Abstand R zu einem Mittelpunkt der Fläche aufweist. Analog dazu ist in den Figuren 2a und 2b eine Gelenkkugel 2 mit einer sphärischen Lagerfläche B dargestellt, die sich über einen Winkel β > 140° erstreckt und die einen Abstand r zum Mittelpunkt $M_K$ der Gelenkkugel aufweist.

Beide Gelenkkugeln in Figur 2a und 2b sind außerhalb der Lagerfläche B durch eine Quasikugelfläche C fortgesetzt, die jedoch einen geringeren Abstand als den Radius r der Lagerfläche B zum Mittelpunkt $M_K$ der Lagerfläche B aufweist. Der kleinere Abstand entsteht beispielsweise dadurch, dass der Gelenkkopf vor dem Schleifen im Hinblick auf den späteren Mittelpunkt $M_K$ im Bereich C bereits abgeplattet oder kegelförmig ausgebildet ist. Die Gelenkkugel ist über eine lösbare Konusverbindung 6 mit einem Prothesenschaft 27 verbunden, wobei die Aufspannachse D vom Konus mit der Rotationsachse für die rotationssymmetrische Lagerfläche B zusammenfällt, damit die Lagerfläche B unabhängig von der konischen Befestigung immer die gleiche Lage einnimmt.

In Figur 3 ist für die sphärischen Flächen A und B jeweils in abgewickelter Form ein Tastschnitt über die Oberfläche dargestellt. Ausgehend von einer nicht gezeigten gemeinsamen Grundlinie ist in einem mittleren Abstand $R_m$ die Fläche A über einen Winkel $\alpha$ und in einem mittleren Abstand $r_m$ die Fläche B über einen Winkel $\beta$ aufgezeichnet. Dabei ist die Vergrößerung senkrecht zur Abtastrichtung, um mehrere Zehnerpotenzen größer als die Vergrößerung in Abtastrichtung dargestellt. Der zulässige Formfehler für die Fläche A der Lagerschale zum mittleren Radius $R_m$ liegt in einer Bandbreite von plus/minus 7,5 $\mu$m und die Rauhigkeit beträgt $R_a < 0,05$ $\mu$m. Für die Fläche B der Kugel beträgt der zulässige Formfehler zum mittleren Radius rm plus/minus 2 $\mu$m und die Rauhigkeit $R_a < 0,05$ $\mu$m. Wenn zu dieser Kombination die Differenz der mittleren Radien in den Grenzen 35 $\mu$m $< R_m-r_m < 85$ $\mu$m liegt und als Werkstoff für die Lagerschale 1 und den Gelenkkopf 2 eine verschleißfeste Metall-Legierung vorliegt, die beispielsweise Blockkarbide als Stützkörper eingelagert hat, dann werden im Beisein von Körperflüssigkeit Tragzahlen erreicht, die trotz der gleichartigen Metall-Legierungen Mikroverschweißungen und Zerstörungen der Oberflächen für die normalen Belastungen an einem Hüftgelenk weitgehend ausschließen.

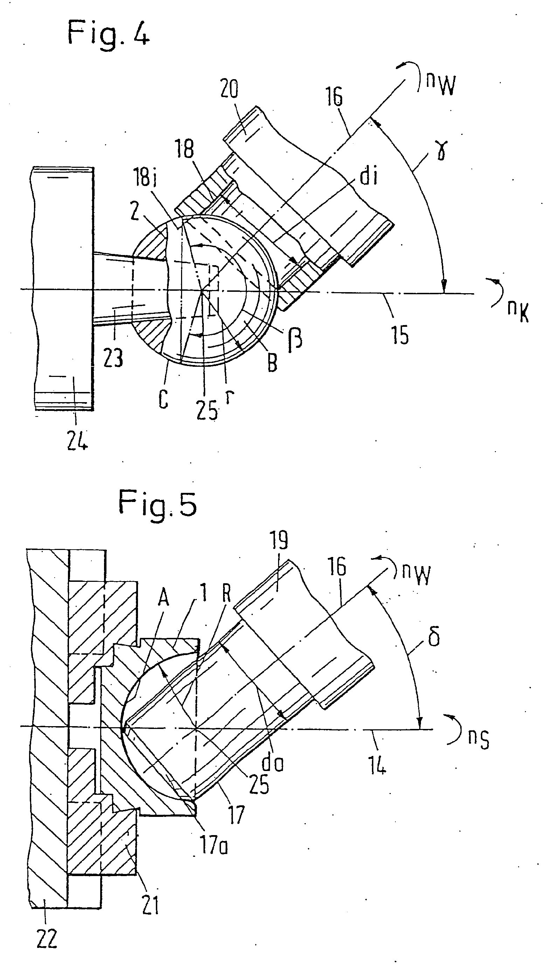

Die Anordnung in Figur 4 betrifft eine Gelenkkugel 2, die mit einem Innenkonus auf einem Aufspanndorn 23 befestigt ist, wobei der Dorn 23 zu einer Werkzeugmaschinenspindel 24 gehört und mit einer Drehzahl $n_K$ von beispielsweise 850 U/min. um seine Rotationsachse 15 dreht. Eine um einen Winkel $\gamma$ von beispielsweise 30° ausgelenkte Werkzeugspindel 20 dreht um ihre Rotationsachse 16 mit einer Drehzahl von beispielsweise 2 000 U/min., wobei sich die beiden Rotationsachsen 15, 16 in einem Schnittpunkt 25 schneiden, der den Mittelpunkt für die später fertig bearbeitete Gelenkkugel 2 bildet. In der Werkzeugspindel 20 ist koaxial ein kreisförmiger Hohlzylinder 18 als Bearbeitungswerkzeug zum Schleifen, Honen oder Polieren eingespannt, welcher mit einer kreisförmigen gebrochenen Innenkante 18i eine Erzeugende mit einem Innendurchmesser di bildet. Der Hohlzylinder besteht aus den üblichen Werkstoffen für Schleifen ohne gebundenes Korn wie z.B. aus Metalloxiden oder Karbiden. Durch Zusetzen eines nicht gezeigten Schleifmittels und Anpressen der Stirnseite des Hohlzylinders 18 in der Richtung der Vorschub- und Rotationsachse 16 schleifen sich die erzeugende Fläche 18i und die Lagerfläche B gegenseitig zu perfekten Ausschnitten von Kugelflächen ein, wobei sich der Radius r der Lagerfläche B ganz langsam verringert und die Erzeugende 18i zu einem kreisförmigen, kugelförmig gewölbten Band vergrößert wird. Durch entsprechende Materialwahl kann der Verschleiß an diesem Band klein gehalten werden. Die so entstehende Lagerfläche B lässt sich durch einen zugehörigen Begrenzungswinkel $\beta$ definieren und hängt in ihrer Größe vom Auslenkwinkel $\gamma$ des Werkzeugs und vom Durchmesser di der Erzeugenden ab. Dementsprechend sind Lagerflächen B mit einem Begrenzungswinkel $\beta$ größer und kleiner 180° möglich, wie in den Figuren 2a, 2b angedeutet ist. Bei einem

Begrenzungswinkel $\beta > 180°$, wie in Figur 4, kann die Abnahme vom Radius r während dem Schleifen durch eine Durchmessermessung über den Schnittpunkt 25 der beiden Rotationsachsen 15, 16 festgestellt werden, um den Abbruch des Schleifens für das Fertigmaß vom Radius r über eine extrapolierbare Zeit festzulegen. Für den Auslenkwinkel $\gamma$ und für den Innendurchmesser di lässt sich als Vorzugsbereich 20° $\leq \gamma \leq 60°$ und 1,8 r $>$ $d_i$ $>$ 1,1 r angeben.

In der Anordnung nach Figur 5 ist eine Lagerschale 1 in einer Werkzeugmaschinenspindel 22 mit einem Spannfutter 21 derart eingespannt, dass die Polachse der Lagerschale 1 und die Rotationsachse 14 der Spindel 22 zusammenfallen, wobei die Spindel 22 mit einer Drehzahl ns von beispielsweise 850 U/min. dreht. Eine Werkzeugspindel 19 ist um einen Auslenkwinkel $\delta$ ausgelenkt und schneidet mit ihrer Rotationsachse 16 die Rotationsachse 14 der Werkstückspindel 22 in einem Schnittpunkt 25, der dem Mittelpunkt der späteren Lagerfläche A entspricht. In die Werkzeugspindel ist koaxial als Werkzeug zum Schleifen ohne gebundenes Korn ein kreisförmiger Vollzylinder 17 eingespannt der mit einer Drehzahl von beispielsweise 2 050 U/min. dreht und mit einer Aussenkante seiner Stirnfläche eine kreisförmige Erzeugende 17a bildet. Durch Zugabe von Schleifmittel und Nachstellen der Erzeugenden 17 in der Richtung der Rotationsachse 16 des Werkzeugs schleifen sich Lagerfläche A und Erzeugende 17a zwangsläufig zu perfekten Ausschnitten von Kugelflächen ein. Um das Maß für den Radius R besser in Griff zu bekommen, wird die Kante 17 vorsorglich zu einer Kugelform abgerichtet. Dies hat den Vorteil, dass der Verschleiß am Werkzeug nur geringfügige Maßänderungen verursacht und dass das Einhalten von einem vorgegebenen Radius R an der Lagerschale 1 erleichtert wird. Für den Auslenkwinkel $\delta$ ergibt sich ein Vorzugsbereich 39° $< \delta < 45°$. Wenn der Begrenzungswinkel $\alpha$ für die Lagerfläche nicht zu weit unter 180° liegen soll und zum Beispiel nur ein Vorschub in der Richtung der Werkzeugrotationsachse 16 erfolgen soll, ergibt sich für den Außendurchmesser $d_a$ der Erzeugenden und den Auslenkwinkel $\delta$ ein bevorzugter Anwendungsbereich

$$1,6\ R < \frac{d_a}{\cos\delta} < 2,2\ R.$$

Die Darstellung in Figur 3 dient dazu, die prinzipiellen Zusammenhänge über die Maßunterschiede der mittleren Radien $R_m$, $r_m$ sowie über die Toleranz 12 für den Formfehler der Lagerschale und die Toleranz 13 für den Formfehler der Gelenkkugel und über die Rauhigkeit der Flächen A, B aufzuzeigen. In der Praxis wird die Qualität der Gelenkkugeln 2 durch Rundheitsmessungen mit einem "Talyround"-Meßgerät überwacht, während die Lagerschalen auf einer Messmaschine auf ihrer Innenseite in verschiedenen Ebenen radial mit einem Messfühler angefahren werden, um aus den Messpunkten eine mittlere Kugelform zu bestimmen und die Formabweichungen zu interpolieren. Der zulässige Formfehler für eine Gelenkkugel 2 im Bereich der Lagerfläche B beträgt plus/minus 2 $\mu$m, während für die Lagerfläche A der Lagerschale ein Formfehler von plus/minus 7,5 $\mu$m zulässig ist. Bei beiden Teilen liegt die Rauhigkeit $R_a$ unter 0,08 $\mu$m, vorzugsweise unter 0,05 $\mu$m.

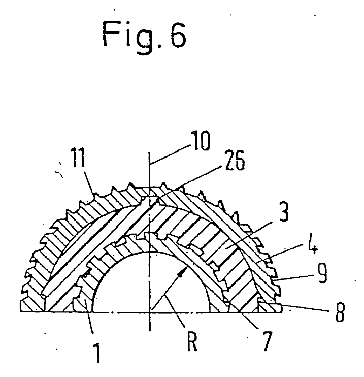

Die Tragfähigkeit dieser fast verschleißfreien Anordnung ist so günstig, dass der Radius für den Gelenkkopf und für die Lagerschale, wie in Figur 6 angedeutet, kleiner als der einer üblichen Polyäthylenlagerschale ausgeführt werden kann. Das heißt, das Auswechseln von Lagerschale und Gelenkkopf ist auch bei implantierten äußeren Schalen 4 möglich, die eine auswechselbare Polyäthylenschale aufweisen, weil die metallische Lagerschale 1 mit einem Zwischenkörper 3 von ausreichender Wandstärke aus Polyäthylen versehen werden kann, dessen äußere Abmessungen denen der ursprünglichen Polyäthylenschale entsprechen. Dabei kann der fest mit der Lagerschale 1 über eine Verbindung 7 verbundene Zwischenkörper 3 eine etwas geringere Wandstärke als die ursprüngliche Polyäthylenlagerschale aufweisen. Dies hat den Vorteil, dass implantierte auswechselbare Polyäthylenschalen durch eine entsprechende Metallschale mit Polyäthylenzwischenkörper ersetzt werden können. Die Außenschale 4 in Figur 6 besitzt bis auf zwei Drittel der Höhe ihrer Außenseite eine Zahnung 9, die zum Äquator deutet, während im oberen Drittel der Außenseite Spitzen 11 angebracht sind, welche parallel zur Polachse 10 ausgerichtet sind. Eine solche Außenschale ist in eine mit Untermass vorbereitete Knochenaushöhlung einschlagbar. Dabei können die Spitzen 11 in ein nicht gezeigtes Knochenbett eindringen, während die abwärts gerichtete Zahnung am Knochenbett unter Vorspannung vorbei gleiten kann und in einer Endlage ein Rückwärtsgleiten mit den Zahnspitzen verhindert, um eine ausreichende Primärverankerung zu erreichen.

**Patentansprüche**

1. Lagerschale (1) für ein künstliches Gelenk, insbesondere künstliches Hüftgelenk, die eine konkave sphärische Fläche A mit Mittelpunkt Ms aufweist,
**dadurch gekennzeichnet,**
**dass** die Lagerschale (1) aus einem verschleißfesten metallischen Werkstoff besteht, und
**dass** der Formfehler der Fläche A über einen Winkel $90° < \alpha < 180°$ weniger als plus/minus 7,5 $\mu$m beträgt,
während die Rauhigkeit der Fläche A einem Wert $R_a < 0,08$ $\mu$m entspricht.

2. Lagerschale nach Anspruch 1,
**dadurch gekennzeichnet,**
**dass** die Rauhigkeit der Fläche A einen Wert $R_a < 0,05$ $\mu$m aufweist.

3. Lagerschale nach Anspruch 1 oder 2,
**dadurch gekennzeichnet,**
**dass** die Lagerschale (1) auf ihrer Außenseite mit einem Zwischenkörper (3) über eine Verbindung (7) fest verbunden ist, wobei der Zwischenkörper mit einer äußeren Schale (4) lösbar verbindbar ist.

4. Gelenkkugel (2) für ein künstliches Gelenk, insbesondere künstliches Hüftgelenk, die eine konvexe sphärische Fläche B mit Mittelpunkt $M_K$ aufweist,
**dadurch gekennzeichnet,**
**dass** die Gelenkkugel (2) aus einem verschleißfesten metallischen Werkstoff besteht,
**dass** der Formfehler der Fläche B über einen Winkel $\beta > 140°$ weniger als plus/minus 2 $\mu$m beträgt, und
**dass** die Gelenkkugel (2) außerhalb der Fläche B durch eine zurückgesetzte Fläche C fortgesetzt ist, die einen geringeren Abstand zum Mittelpunkt $M_K$ als die Fläche B aufweist,
während die Rauhigkeit der Fläche B einem Wert $R_a < 0,08$ $\mu$m entspricht.

5. Gelenkkugel nach Anspruch 4,
**dadurch gekennzeichnet,**
**dass** die Rauhigkeit der Fläche B einen Wert $R_a < 0,05$ $\mu$m aufweist.

6. Gelenkkugel nach Anspruch 4 oder 5,
**dadurch gekennzeichnet,**
**dass** die Gelenkkugel über eine lösbare Konusverbindung (6) in der Richtung der Aufspannachse D mit dem Schaft verbindbar ist, um unabhängig von der Verbindung eine gleich wirkende Teilkugelfläche B zu erhalten.

7. Gelenkkugel nach einem der Ansprüche 4 bis 6,
**dadurch gekennzeichnet,**
**dass** die Fläche B rotationssymmetrisch zu einer Aufspannachse D in der Richtung des Schenkelhalses eines künstlichen Hüftgelenkschaftes (27) angeordnet ist.

## Fig.1

## Fig.2a

## Fig.2b

## Fig.3

Fig. 4

Fig. 5

8

# Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9716138 A1

**In der Beschreibung aufgeführte Nicht-Patentliteratur**

- **STREICHER.** Untersuchung des tribologischen Verhaltens von Metall/Metall Kombinationen für künstliche Hüftgelenke. *BIOMEDIZINISCHE TECHNIK,* 1990, vol. 35 (5), 107-111